# Europäisches Patentamt

## European Patent Office

## Office européen des brevets

(19)

(11) Veröffentlichungsnummer: **0 006 671**
**B1**

(12)
# EUROPÄISCHE PATENTSCHRIFT

(45) Veröffentlichungstag der Patentschrift:
**15.09.82**

(51) Int. Cl.³: **C 12 N 1/04**

(21) Anmeldenummer: **79200338.6**

(22) Anmeldetag: **26.06.79**

(54) Verfahren zur Erhaltung lebender Mikroorganismen.

(30) Priorität: **03.07.78 CH 7218/78**

(43) Veröffentlichungstag der Anmeldung:
**09.01.80 Patentblatt 80/1**

(45) Bekanntmachung des Hinweises auf die Patenterteilung:
**15.09.82 Patentblatt 82/37**

(84) Benannte Vertragsstaaten:
**AT CH DE FR GB IT**

(56) Entgegenhaltungen:
**BE-A-665 076**
**CH-A-339 326**
**DE-A-1 642 604**
**DE-B-481 614**
**FR-A-2 346 445**
**GB-A-27 711**
**GB-A-408 362**
**GB-A-484 818**
**US-A-1 386 359**

(73) Patentinhaber: **Firma Dr.Ing. Hans Müller, Alte Landstrasse 415, CH-8708 Männedorf (CH)**

(72) Erfinder: **Müller, Hans, Dr. Ing., Im Allmendli, CH-8703 Erlenbach (CH)**

(74) Vertreter: **Herrmann, Peter, Firma Chemap AG Alte Landstrasse 415, CH-8708 Männedorf (CH)**

**US-A-2 213 283**
**US-A-2 710 810**
**US-A-3 075 887**
**CHEMICAL ABSTRACTS, Band 80, 1974, Seite 317, Spalte 2, Zusammenfassung 25 905 p. Columbus, Ohio, USA**
**CHEMICAL ABSTRACTS, Band 78, Nr. 5, 5. Februar 1973, Seite 364, Spalte 2, Zusammenfassung 27 943 g. Columbus, Ohio, USA**

## Verfahren zur Erhaltung lebender Mikroorganismen

Die Erfindung betrifft ein Verfahren zur Erhaltung und Präservierung von Reinkulturen lebender Mikroorganismen, insbesondere Kulturen von Hefen und Bakterien.

Zur Aufbewahrung von lebenden Reinkulturen genügt nicht allein die Erhaltung von Mikroorganismen, sondern es müssen auch deren Eigenschaften konserviert werden.

Verfahren zur Erhaltung von Mikroorganismen sind bekannt. Für die langfristige Aufbewahrung, sowohl von Aerobiern als auch von Anaerobiern, haben sich die Verfahren der Gefriertrocknung, der Tiefkühlung mit Glyzerin und der Ultratiefkühlung in flüssigem Stickstoff als geeignet erwiesen. Für kürzerfristige Aufbewahrung von einigen Monaten ist der Schrägagar mit ca. 30% Wassergehalt geeignet und für einige Tage der Schüttelkolben zur Aufbewahrung von Mikroorganismen in einer Nährlösung im Kühlschrank.

Es sind aber auch Verfahren durch Heißtrocknung von Mikroorganismen, unter Verwendung von organischen Trägerstoffen, die während der Trocknung zugesetzt werden, bekannt.

So beschreibt die CH-A-339 326 ein Verfahren zur Herstellung von Bazillen-Trockenkulturen. Die suspendierte Biomasse wird nach dem Abzentrifugieren der Nährlösung mit 0,3 bis 1% Gelatine als Dickungsmittel vor der Trocknung emulgiert. Die Trocknung erfolgt im Heißluftstrom bei 100 − 200° C.

Aus der US-A-1 386 359 ist ein Verfahren zur Herstellung von Trockenbackhefe bekannt. Es werden Stärkemehl und Glucose als organisches Füllmaterial während der Trocknung zugegeben, um den Start der Gärung bei der Teigbereitung zu beschleunigen.

Alle diese Methoden weisen jedoch Nachteile auf. So ist die Gefriertrocknung sehr aufwendig und teuer und nur als Aufbewahrung von Kulturen in Sammlungen wirtschaftlich vertretbar. Die Zugabe organischer Trägerstoffe während der Trocknung begünstigt eine Klumpenbildung, erhöht das Risiko einer chemischen Reaktion dieser Stoffe während der Trocknung. Besondere Schwierigkeiten bereitet jedoch ihr physikalisches Verhalten beim Wiederaufschmelzen.

Auch treten beim Transport von Reinkulturen in entferntere Gebiete große Probleme auf. Oft kann eine Reinzucht nicht an Ort und Stelle vorgenommen werden, entweder fehlt die dafür notwendige Ausrüstung oder das geschulte Personal. Beispielsweise braucht die Herstellung von Backhefe an sich nicht unter sterilen Bedingungen zu erfolgen, da die kontinuierliche Züchtung wegen der Abnahme der Triebkraft noch nicht durchführbar ist. Eine Reinzuchthefe jedoch muß unter sterilen Bedingungen durchgeführt werden. Die Herstellung von Essigsäure ist vor allem deshalb sehr schwierig, da bei Luftausfall von wenigen Minuten eine Abtötung der größten Menge der dafür notwendigen Bakterien eintritt. Hier muß immer eine Reinkultur in größeren Mengen zur Wiederanzüchtung möglichst rasch zur Verfügung stehen. Ein Transport über weitere Entfernungen kann kaum durchgeführt werden, da die dauernde sterile Belüftung während des Transportes gewährleistet sein müßte.

Aufgabe der Erfindung ist es, ein Verfahren zu schaffen, das Reinzuchtkulturen so zu präservieren vermag, daß eine Aufbewahrung über längere Zeit möglich ist.

Diese Aufgabe wird erfindungsgemäß nach Patentanspruch 1 gelöst und ist dadurch gekennzeichnet, daß die lebenden Mikroorganismen in homogener Suspension zusammen mit einem zur Konzentrierung verwendeten Filterhilfsmittel unter sterilen Bedingungen filtriert und zu einem Granulat getrocknet werden.

Die Granulatform hat sich als besonders vorteilhaft erwiesen, da damit eine Abfüllung der Partikel leicht möglich ist und durch die große Oberfläche die Auflösung bei der Wiederanzucht rasch erfolgen kann.

Die Erfindung soll anhand eines Beispiels näher erläutert werden, ohne jedoch auf die Mikroorganismen des Beispiels beschränkt zu werden.

### Beispiel

In 500 ml Wasser werden unter Rühren bei 60 − 80° C 50 g Gelatine gelöst, in bekannter Weise sterilisiert und auf 30 − 35° C abgekühlt. Während der Abkühlung wird gerührt, damit eine gleichmäßige Temperaturverteilung erfolgt. Nun werden unter sterilen Bedingungen 50 cm$^3$ einer 10 − 15%igen Hefemilch, die lebende Zellen enthält, zugegeben. Die Trocknung wurde mit steriler Luft von 40° C im Laborsprühtrockner durchgeführt. Das so getrocknete Gelatine-/Reinkulturgranulat kann bei Raumtemperatur in sterilen Verpackungen gelagert werden. Nach Wiederaufschmelzen bei 30 − 35° C in einer Nährlösung tritt rasches Wachstum auf. Der Anteil an toten Zellen war unter 5%.

Zur Aufkonzentrierung wird vor der Trocknung ein Großteil des Wassers durch Filtration mittels Filterhilfsmittel, wie beispielsweise Kieselgur, entfernt. Bei Anwendung dieser Verfahrensweise ist es nicht notwendig, die Mikroorganismen vom Filterhilfsmittel abzutrennen. Die Mikroorganismen können in bekannter Weise zusammen mit dem Filterhilfsmittel getrocknet werden.

Auch kann das zum Wiederwachsen erforderliche Nährmedium vor der Trocknung der Suspension zugemischt werden oder aber nach der Trocknung in festem Zustand zugegeben werden.

Die Trocknung wird vorzugsweise in einem Sprühtrockner unter Vakuum durchgeführt. Jede andere, unter schonenden Bedingungen durchgeführte Trocknung ist auch zur Erzielung des erfindungsgemäßen Produktes geeignet. Es ist jedoch darauf zu achten, daß der optimale Temperaturbereich während der Trocknung 10–50° C am Produkt beträgt und vorzugsweise 40° C nicht übersteigt.

Als Hefe können alle Arten von Kulturhefen, wie beispielsweise Saccharomyces cerevisiae und Bakterien, wie beispielsweise Essigsäurebakterien nach dem erfindungsgemäßen Verfahren konserviert werden.

Die Trocknung wird bis auf einen Restwassergehalt von 1–15% je nach Konfektionierung und Lagermöglichkeiten eingestellt.

Das erfindungsgemäße Verfahren hat den Vorteil, daß auch eine konzentrierte Mikroorganismen-Reinkultur auf einfachem und wirtschaftlichem Wege hergestellt werden kann. Zum Transport und zur Lagerung sind nur kleine Räume erforderlich. Die Wiederanzucht einer so hergestellten Kultur geht rasch und problemlos vor sich.

## Patentansprüche

1. Verfahren zur Erhaltung und Präservierung von Reinkulturen lebender Mikroorganismen in Gegenwart einer hochmolekularen, quellfähigen, wasserlöslichen Trägersubstanz, dadurch gekennzeichnet, daß die lebenden Mikroorganismen in homogener Suspension zusammen mit einem zur Konzentrierung verwendeten Filterhilfsmittel unter sterilen Bedingungen filtriert und zu einem Granulat getrocknet werden.

2. Verfahren nach Anspruch 1, dadurch gekennzeichnet, daß als Trägersubstanz Gelatine verwendet wird.

3. Verfahren nach den Ansprüchen 1 und 2, dadurch gekennzeichnet, daß als Mikroorganismen Bakterien verwendet werden.

4. Verfahren nach den Ansprüchen 1 bis 3, dadurch gekennzeichnet, daß die Trocknung zwischen 10 und 50° C vorgenommen wird.

## Claims

1. A process for the storage and preservation of pure cultures of viable microorganisms, in the presence of a carrier material of high molecular weight, swellable and watersoluble, which is characterised by the fact that the living microorganisms, kept in a homogeneous suspension, together with filteraid material, used for concentration, can be filtered under sterile conditions and can then be dried to form a granular material.

2. A process, according to claim 1, characterised by the use of gelatine as the carrier substance.

3. A process, according to claims 1 and 2, characterised by the use of bacteria as microorganisms.

4. A process, according to claims 1 to 3, characterised by the drying step, which is performed in the range of 10° to 50° C.

## Revendications

1. Procédé d'obtention et de conservation de culture pure de microorganismes vivants en présence d'une substance support d'un poids moléculaire élevé, susceptible de gonfler et soluble dans l'eau, caractérisé en ce que les microorganismes vivants en suspension homogène dont la suspension contient en même temps un adjuvant de filtration pour la concentration, sont filtrés sous conditions stériles et amenée par séchage sous la forme de granulés.

2. Procédé selon la revendication 1, caractérisé en ce que la gélatine est utilisée à titre de support. .

3. Procédé selon les revendications 1 et 2, caractérisé en ce que les microorganismes sont des bactéries.

4. Procédé selon les revendications 1 à 3, caractérisé en ce que le séchage est effectué entre 10 à 50° C.